# FASCICULE DE BREVET EUROPEEN

(11) **EP 3 438 129 B1**
(45) Date de publication et mention de la délivrance du brevet: **22.07.2020**
(21) Numéro de dépôt: 18192072.9
(22) Date de dépôt: 12.12.2011
(51) Int. Cl.: C07K 16/28, A61K 39/395, A61P 35/00, A61P 37/00

(54) **COMPOSITION D'ANTICORPS MONOCLONAUX DIRIGES CONTRE BDCA-2**
ZUSAMMENSETZUNG AUS MONOKLONALEN ANTIKÖRPERN, DIE GEGEN BDCA-2
COMPOSITION OF MONOCLONAL ANTIBODIES DIRECTED AGAINST BDCA-2

(30) Priorité: 13.12.2010 FR 1060428
(43) Date de publication de la demande: 06.02.2019
(62) Demande divisionnaire de: 17159253.8
(73) Titulaire: Laboratoire Français du Fractionnement et des Biotechnologies, 91940 Les Ulis (FR)
(72) Inventeur: FOURNIER, Nathalie, 59130 Erquinghem-Lys (FR); DE ROMEUF, Christophe, 59130 Lambersart (FR)
(74) Mandataire: Plasseraud IP

(56) Documents cités:
- WO-A1-2006/037247
- WO-A2-01/36487
- NESTLE F O ET AL: "Plasmocytoid predendritic cells initiate psoriasis through interferon-alpha production", THE JOURNAL OF EXPERIMENTAL MEDICINE, ROCKEFELLER UNIVERSITY PRESS, US, vol. 202, no. 1, 4 juillet 2005 (2005-07-04), pages 135-143, XP002364226, ISSN: 0022-1007, DOI: DOI:10.1084/JEM.20050500
- BLOMBERG S ET AL: "Expression of the markers BDCA-2 and BDCA-4 and production of interferon-alpha by plasmacytoid dendritic cells in systemic lupus erythematosus", ARTHRITIS & RHEUMATISM, JOHN WILEY & SONS, INC, US, vol. 48, no. 9, 1 septembre 2003 (2003-09-01), pages 2524-32, XP002991721, ISSN: 0004-3591, DOI: DOI:10.1002/ART.11225
- JAHN P S ET AL: "BDCA-2 signaling inhibits TLR-9-agonist-induced plasmacytoid dendritic cell activation and antigen presentation", CELLULAR IMMUNOLOGY, ACADEMIC PRESS, SAN DIEGO, CA, US, vol. 265, no. 1, 1 janvier 2010 (2010-01-01), pages 15-22, XP027236570, ISSN: 0008-8749 [extrait le 2010-07-06]
- DZIONEK A ET AL: "BDCA-2, a novel plasmacytoid dendritic cell-specific type II C-type lectin, mediates antigen capture and is a potent inhibitor of interferon alpha/beta induction", THE JOURNAL OF EXPERIMENTAL MEDICINE, ROCKEFELLER UNIVERSITY PRESS, US, vol. 194, no. 12, 17 décembre 2001 (2001-12-17), pages 1823-1834, XP002277387, ISSN: 0022-1007, DOI: 10.1084/JEM.194.12.1823
- CHARLES ET AL: "Plasmacytoid dendritic cells and dermatological disorders: focus on their role in autoimmunity and cancer", EUR J DERMATOL, vol. 20, no. 1, 1 janvier 2010 (2010-01-01), pages 1-8, XP055463394,
- JAYE DAVID L ET AL: "Expression of the plasmacytoid dendritic cell marker BDCA-2 supports a spectrum of maturation among CD4+CD56+hematodermic neoplasms", MODERN PATHOLOGY, vol. 19, no. 12, 1 septembre 2006 (2006-09-01), pages 1555-1562, XP002481366, NATURE PUBLISHING GROUP, US ISSN: 0893-3952, DOI: 10.1038/MODPATHOL.3800679
- LONARDI S ET AL: "A monoclonal antibody anti-BDCA2 (CD303) highly specific for plasmacytoid dendritic cell neoplasms on paraffin sections", VIRCHOWS ARCHIV, vol. 455, no. Suppl. 1, août 2009 (2009-08), pages 254-255, XP002644181, & 22ND EUROPEAN CONGRESS OF PATHOLOGY; FLORENCE, ITALY; SEPTEMBER 04 09, 2009 ISSN: 0945-6317

## Description

La présente invention a pour objet l'utilisation d'un anticorps dirigé contre une protéine membranaire, et notamment pour le traitement de pathologies.

Les anticorps utilisés en clinique, dans le but de réguler une pathologie auto-immune ou inflammatoire, sont à ce jour principalement les anticorps anti-CD20 et en particulier l'anti-CD20 Rituxan. Le problème associé à ce type de traitement est dû au fait que les anti-CD20 vont éliminer l'ensemble des cellules pré-B et lymphocytes B matures, mais également environ 20% des plasmocytes et une population de lymphocytes B mémoire (Gonzales-Stawinski Clin Immunol 2001, 98, 175 ; Looney, Ann rheum dis 2002, 61, 863) pouvant ainsi conduire à un état immuno-déficient associé à une hypogammaglobulémie.

Il existe donc un besoin de trouver une alternative à cette déplétion B en diminuant le nombre de cellules dendritiques plasmacytoïdes, cellules clés à l'initiation de la réponse immune pathologique, et donc atténuer la réponse immune du patient et par conséquence de cellules B auto-réactives.

Les cellules dendritiques (en anglais dendritic cells ou DC) sont des cellules du système immunitaire présentatrices d'antigènes (CPA) faisant partie du système réticulohistiocytaire. Sous certaines conditions, les cellules DC présentent des prolongements cytoplasmiques similaires aux dendrites des neurones.
Les cellules dendritiques ont deux fonctions principales :
- le déclenchement de la réponse immunitaire adaptative, dont les acteurs principaux sont les lymphocytes T et les lymphocytes B, dirigée contre des antigènes du « non-soi ».
- le maintien de la tolérance centrale au « soi » dans le thymus, par le processus impliquant les lymphocytes T dit de sélection négative.

De nombreuses sous populations de cellules DC ont été caractérisées chez la souris, sans pour autant qu'il ait été identifié de correspondance chez l'homme.
Les DC sont capables de se différencier en divers sous-types, selon les stimuli qu'elles reçoivent. Il existe deux grands types de cellules DC : les cellules DC conventionnelles, les cellules DC plasmacytoïdes et les cellules DC inflammatoires.
Les cellules DC conventionnelles comprennent :
- les cellules DC migratoires qui résident, à l'état basal, en périphérie. Suite à la phagocytose d'une particule antigénique et/ou à la réception de signaux, elles migrent vers les ganglions lymphoïdes secondaires par l'intermédiaire des vaisseaux lymphatiques ; elles arrivent à l'état mature dans les organes lymphoïdes où elles présentent l'antigène aux lymphocytes T naïfs ; on peut citer par exemple des cellules de Langerhans ou encore les cellules D des muqueuses ;
- les cellules DC immatures résidentes des organes lymphoïdes ; ces cellules collectent et présentent les antigènes du soi ou étrangers au sein même des organes lymphoïdes ; il s'agit de la plupart des cellules DC du thymus ou de la rate.

Les cellules DC inflammatoires sont recrutées dans les tissus suite à une inflammation où une infection mais ne sont jamais présentes en dehors de toute stimulation. On suppose actuellement que les cellules DC inflammatoires seraient principalement issues de la différenciation des monocytes sanguins.

Les cellules DC plasmacytoïdes diffèrent des cellules DC conventionnelles en ce qu'elles sont circulantes, rondes et sans dendrites à l'état basal, bien qu'elles finissent par se différencier en DC conventionnelles après activation. Elles sont donc capables de présenter l'antigène.
Après stimulation, en général par un antigène viral, elles produisent une grande quantité d'interférons (IFN) de classe I. Ces cellules sont essentiellement impliquées dans la réponse anti-virale et dans les désordres auto-immuns.

Les cellules DC plasmacytoïdes (aussi appelées cellules DC plasmacytoïdes) ont été caractérisées phénotypiquement : elles expriment les marqueurs CD4 et BDCA-2 et 4. Leur phénotype est donc CD4+, CD11c-, Lin-, BDCA-2+, BDCA-4+.

Les cellules DC plasmacytoïdes peuvent être à l'origine de tumeurs hématopoïétiques dans lesquelles elles acquièrent un marqueur supplémentaire qui est le CD56+. C'est pourquoi on parle de tumeurs hématopoïétiques CD4+/CD56+ (en anglais BPDCN : Blastic plasmacytoid dendritic cells neoplasm).

Les tumeurs hématopoïétiques CD4+/CD56+ sont des néoplasmes hématologiques rares (1% des leucémies aiguës), qui se présentent sous la forme de nodules cutanés associés à une lympho-adénopathie ou un gonflement de la rate et à une cytopénie fréquente. Ces manifestations cutanées sont très rapidement suivies par une infiltration de la moelle osseuse. Du fait de l'expression du marqueur CD56+, ces pathologies ont d'abord été classées dans les lymphomes à cellules NK. Mais l'absence de marqueurs de lignées myéloïdes, lymphoïdes T ou B ou NK a suscité des études complémentaires qui ont permis d'affiner la classification pour enfin révéler que les cellules dendritiques dite de type II ou cellules DC plasmacytoïdes sont à l'origine des tumeurs hématopoïétiques CD4+/CD56+ [Chaperot L et al., 2001, Blood. 2001 May 15;97(10):3210-7*,* Herling M, Jones D., 2007, Am J Clin Pathol. 2007 May;127(5):687-700]

La prise en charge actuelle de ces pathologies est basée sur la chimiothérapie qui permet une rémission complète pour 2 patients sur trois environ mais où les rechutes sont fréquentes et précoces (environ 9 mois). La médiane de survie globale est de 13 mois environ. Une autre alternative de traitement est l'allogreffe de cellules hématopoïétiques qui ne permet néanmoins pas non plus de survie à long terme.

En conséquence, il existe à ce jour un réel besoin de trouver un traitement à ces pathologies CD4+/CD56+.

Des moyens de traitement de ces pathologies impliquant des cellules exprimant des marqueurs des cellules dendritiques sont proposés dans l'art antérieur.
Notamment, des molécules ciblant les marqueurs de différentiation BDCA ont été proposées.

On peut citer par exemple le brevet européen EP 1 301 539 qui décrit un polypeptide de séquence particulière se liant spécifiquement à la protéine BDCA-2 et une composition pharmaceutique le comprenant.
La demande de brevet européen EP 1 783 141 divulgue une composition permettant d'induire une réponse immunitaire chez un patient, ladite composition comprenant des cellules dendritiques préalablement traitées avec un anticorps anti-BDCA-2 et exprimant un antigène (tumoral, viral, bactérien...). Ceci consiste en un traitement par thérapie cellulaire.
La demande WO 01/365487 décrit des anticorps monoclonaux dirigés contre la protéine BDCA-2, et en particulier les clones AC144, AD5-13A11 et AD5-4B8, ainsi que des fragments dérivés. Par ailleurs, cette demande décrit l'utilisation des anticorps monoclonaux pour le traitement de pathologies telles que les infections virales, les maladies auto-immunes et les tumeurs.
La demande WO 2006/037247 décrit l'utilisation d'anticorps monoclonaux dirigés contre la protéine BDCA-2, dans le cadre du traitement d'une maladie auto-immune particulière : le psoriasis.
Nestle et al., (Nestle et al. 2005, J. Exp. Med, vol 202, n°1, pp: 135-143) enseigne que des anticorps dirigés contre la protéine BDCA-2 peuvent être injectés par voie intraveineuse, dans un but thérapeutique dans le cadre du traitement du psoriasis.

Blomberg et al., (Blomberg et al., 2003, Arthritis and Rheumatism, vol 48, n°9, pp : 2524-2532) enseigne l'utilisation d'anticorps dirigés contre la protéine BDCA-2, lesdits vaccins étant utilisés pour d'une maladie auto-immune : le Lupus Erythémateux. Les auteurs montrent que lesdits anticorps sont capables d'inhiber la production d'interféron α(IFN-α).
La demande US 2010/1896641 décrit des anticorps dirigés contre la protéine BDCA-2 dans le cadre du traitement des tumeurs.

On constate donc que l'utilisation d'anticorps dirigés contre la protéine BDCA-2 est largement décrite dans l'art antérieur.

Toutefois, l'art antérieur est muet quant au traitement de pathologies impliquant des cellules CD4+/CD56+.

Cependant, à ce jour il n'existe aucun traitement spécifique et efficace permettant le traitement ou la prévention de pathologies impliquant des cellules dendritiques plasmacytoïdes, et notamment les tumeurs CD4+/CD56+.

L'invention a donc pour objet de pallier le manque de l'art antérieur, et notamment de fournir des traitements alternatifs.
De plus, l'invention a pour but de fournir un nouveau traitement efficace et spécifique des tumeurs à cellules CD4+/CD56+.
L'invention a également pour objet des compositions médicamenteuses en vue de traiter et/ou prévenir lesdites pathologies.
L'invention est définie par les revendications.
L'invention concerne un anticorps monoclonal ou polyclonal dirigé contre la protéine BDCA-2 pour son utilisation dans le cadre de la prévention ou du traitement des pathologies impliquant une activation des cellules dendritiques plasmacytoïdes. L'invention repose sur la constatation inattendue faite par les Inventeurs de l'effet d'anticorps anti-BDCA-2 sur la progression de tumeurs, notamment des tumeurs hématopoïétiques.
L'invention se distingue de l'art antérieur en ce qu'elle implique une destruction des cellules DC plasmacytoïdes par apoptose ou par le mécanisme de l'ADCC.

L'utilisation d'anticorps anti-IFN-a dans le traitement des patients atteints de pathologies auto-immunes et inflammatoires induit une neutralisation de l'IFN-a d'une façon systémique, ce qui augmente potentiellement le risque d'infections opportunistes. En effet cette cytokine est produite en réponse à un agent infectieux en particulier viral. Pour répondre à cette problématique, l'objet de l'invention consiste à éliminer les cellules DC plasmacytoïdes, source principale d'IFN-a, recrutées au niveau des lésions inflammatoires et responsable de l'inflammation locale, ceci étant particulièrement bien décrit dans le cas du psoriasis par exemple. L'avantage de l'invention réside donc en une élimination de l'IFN-a préférentiellement au lieu de l'inflammation et non de façon systémique, ceci diminuant par fait les risques liés à l'immunodéficience induits par les anti-IFN-a.
Un avantage certain et additionnel réside en l'absence d'effet sur les leucocytes (Papot, 2002, rev med interne, 23, supp 4), les macrophages (Levesque MC, 1999, Arthritis Rheum, 42, 569) et les fibroblastes (Houglum, JE, 1893, 2,20) et ainsi de préserver les sources d'IFN-a produites par ces types cellulaires.

Un autre avantage consiste *via* l'élimination des cellules DC plasmacytoïdes est la diminution d'autres cytokines pro-inflammatoires comme l'IL-6 également sécrétées par la cellules DC plasmacytoïdes dans un environnement inflammatoire et conduisant à une réponse Th1 par exemple.

De plus, contrairement aux concepts généralement admis, l'objet de l'invention consiste à prendre avantage de l'hyper-activation du système immunitaire au site de l'inflammation, en particulier l'activation des cellules effectrices dont les cellules NK et les macrophages et cela du à la présence de nombreuses cytokines et chimiokines pro-inflammatoires pour obtenir *in fine* une diminution de l'inflammation. En effet, les auteurs ont montré que l'anticorps anti-BDCA-2 cité avait une meilleure activité cytotoxique contre les pDC en présence de molécules dont les taux sont augmentés dans les pathologies auto-immunes et inflammatoires (ie IFN-γ, TNF-α, ...).

La protéine BDCA-2 est exprimée de manière spécifique à la surface des cellules DC plasmacytoïdes, et est une protéine de type II appartenant aux lectines de type C.
Dans le cadre de la présente invention, le terme « anticorps » se réfère à une immunoglobuline, protéine constituée de 4 chaînes participant à la réponse immunitaire acquise

Les immunoglobulines sont bien connues de l'homme de métier et sont constituées d'un assemblage de deux dimères constitués chacun d'une chaîne lourde et d'une chaîne légère. Le complexe multimérique est assemblé par la liaison d'une chaîne légère et d'une chaîne lourde par un pont disulfure entre deux cystéines, les deux chaînes lourdes étant elle-même également reliées entre elles par deux ponts disulfures.
Chacune des chaînes lourdes et des chaînes légères est constituée d'une région constante et d'une région variable. L'assemblage des chaînes qui composent un anticorps permet de définir une structure tridimensionnelle caractéristique en Y, où
- la base du Y correspond à la région constante Fc qui est reconnue par le complément et les récepteurs Fc, et
- l'extrémité des bras du Y correspondent à l'assemblage respectif des régions variables de la chaîne légère et variable de la chaîne lourde qui sont reconnues par un antigène spécifique.

Plus précisément, chaque chaîne légère est constituée d'une région variable (V_{L}) et d'une région constante (C_{L}). Chaque chaîne lourde est constituée d'une région variable (V_{H}) et d'une région constante constituée de trois domaines constants C_{H1}, C_{H2} et C_{H3}. Les domaines C_{H2} et C_{H3} composent le domaine Fc.

Les anticorps décrits dans l'invention sont isolés et purifiés, et sont différents des anticorps naturels. Ces anticorps sont matures, c'est-à-dire qu'ils possèdent une structure tridimensionnelle ad hoc leur permettant de reconnaitre l'antigène, et possèdent toutes les modifications post-traductionnelles essentielles à leur reconnaissance antigénique.
Dans un aspect de l'invention, les anticorps sont polyclonaux.
Dans un autre aspect, il s'agit d'anticorps monoclonaux, c'est-à-dire qu'ils ne reconnaissent qu'un seul déterminant antigénique dans BDCA-2, contrairement aux anticorps polyclonaux qui correspondent à un mélange d'anticorps monoclonaux, et donc peuvent reconnaitre plusieurs déterminants antigéniques dans une même protéine.
Les anticorps monoclonaux de l'invention peuvent être obtenus par des techniques bien connues de l'homme de métier, et notamment par la technique de fusion cellulaire, ou encore la technique de clonage des séquences des chaines lourdes et légères, par technique de phage ou ribosome display, par immunisation de souris ayant le répertoire des immunoglobulines humaines et expression dans une cellule ad hoc ou un animal transgénique.
Ces techniques sont bien connues de l'homme de métier.
Par cellules dendritiques plasmacytoïdes, on entend la sous population de cellules dendritiques aussi appelée DC2 caractérisée par les marqueurs HLA-DR+, CD11c-, CD123+ et CD45RA+, présentes dans les organes lymphoïdes et également en circulation dans le sang et caractérisées par leur capacité à sécréter de l'IFN de type I en présence d'une infection virale.

Dans l'invention, on entend par activation des cellules dendritiques plasmacytoïdes les différents mécanismes ayant pour effet d'activer la prolifération, la sécrétion cytokine déterminées, notamment les interférons de classe I, et la modification phénotypiques et morphologiques des cellules plasmacytoïdes.

Dans un mode de réalisons avantageux, l'invention concerne un anticorps monoclonal ou polyclonal dirigé contre la protéine BDCA-2 pour son utilisation définie précédemment, où lesdites pathologies impliquant des cellules dendritiques plasmacytoïdes sont choisies parmi les tumeurs, les maladies auto-immunes et les maladies inflammatoires.

De manière avantageuse, l'invention concerne un anticorps monoclonal ou polyclonal dirigé contre la protéine BDCA-2 pour son utilisation susmentionnée, où les tumeurs sont de préférences des tumeurs hématopoïétiques.
Dans l'invention, on entend par tumeurs hématopoïétiques des tumeurs ou maladies tumorales impliquant des cellules de la lignée sanguine, ou cellules hématopoïétiques. Ces tumeurs peuvent également être appelées hémopathies malignes.
Les hémopathies malignes embrassent
- les leucémies : tumeur où les cellules sanguines prolifèrent anormalement dans le sang,
- les lymphomes : tumeur cellules sanguines prolifèrent anormalement dans les organes lymphoïdes secondaires (ganglions, rate...)
- ou la Maladie de Kahler : tumeur où les cellules sanguines prolifèrent anormalement la moelle osseuse.

Un autre mode de réalisation avantageux de l'invention concerne un anticorps monoclonal ou polyclonal dirigé contre la protéine BDCA-2 pour son utilisation telle que définie précédemment, où les tumeurs hématopoïétiques sont des tumeurs de phénotype CD4+, CD56+.

On entend par tumeurs hématopoïétiques CD4+/CD56+, des hémopathies malignes dans lesquelles les cellules cancéreuse expriment conjointement les deux marqueurs CD4 et CD56. Ces maladies embrassent les lymphomes cutanés agranulaire CD4+/CD56+, les hématodermies agranulaires CD4+/CD56+, les leucémies/lymphomes NK blastiques, les lymphomes NK [Ng AP et al. 2006. Haematologica 91 (1): 143-4*;*Kim Y et al. 2005 J. Korean Med. Sci. 20 (2): 319-24 *;* Chan JK, et al. 1997 Blood 89 (12): 4501-13].

Dans un autre mode de réalisation avantageux, l'invention concerne un anticorps monoclonal ou polyclonal dirigé contre la protéine BDCA-2 pour son utilisation telle que définie ci-dessus, où les maladies auto-immunes et inflammatoires sont choisies parmi la maladie des tissus conjonctifs, les différents type de scléroses, l'inflammation autoimmune pulmonaire, le syndrome Guillain-Barre, la thyroidite autoimmune, le mellitis, le myasthenia gravis, la maladie du greffon contre l'hôte, la maladie autoimmune inflammatoire de l'œil, le psoriasis, la maladie de Basedow (hyperthyroïdie), la thyroïdite chronique de Hashimoto (hypothyroïdie), le lupus érythémateux disséminé (LED), le syndrome de Goodpasture, le pemphigus, la Myasthénie, le diabète par insulinorésistance, l'anémie hémolytique auto-immune, le purpura thrombocytopénique auto-immun, la polyarthrite rhumatoïde, la sclérodermie, polymyosite et dermatomyosite, l'anémie de Biermer, la maladie de Gougerot-Sjogren, la glomérulonéphrite, la maladie de Wegener, la maladie de Horton, la périartérite noueuse et le syndrome de Churg et Strauss, la maladie de Still, la polychondrite atrophiante, la maladie de Behçet, la sclérose en plaques, la spondylarthrite, la maladie de Crohn, la gammapathie monoclonale, la granulomatose de Wegener, le lupus, la maladie de Horton, la maladie de Reiter, la rectocolite hémorragique, le rhumatisme psoriasique, la sarcoïdose, la spondylarthrite ankylosante, les dermatites bulleuses auto-immunes, la colite collagène, dermatite herpétiforme, fièvre méditerranéenne familiale, glomérulonéphrite à dépôts d'IgA, la glomérulonéphrite extra-membraneuse, l'hépatite auto-immune, le syndrome myasthénique de Lambert-Eaton, l'ophtalmie sympathique, la pemphigoïde bulleuse, le pemphigus, le purpura thrombopénique idiopathique, le syndrome de Fiessinger-Leroy-Reiter, la thyroïdite auto-immune, le syndrome uvéo-méningo-encéphalique et la dermatite herpétiforme.

Dans un autre mode de réalisation préféré de l'invention, les pathologies impliquant des cellules dendritiques plasmacytoïdes sont des maladies auto-immunes, de préférence des maladies auto-immunes caractérisées par une sécrétion accrue d'IFN de type I.

Dans un mode de réalisation avantageux, l'invention concerne un anticorps monoclonal ou polyclonal dirigé contre la protéine BDCA-2 pour son utilisation selon la définition précédente, où les tumeurs sont des tumeurs hématopoïétiques sont choisies parmi syndromes lymphoprolifératifs et myélome (maladie de Waldenström, leucémie lymphoïde chronique, myélome multiple, amylose primitive), les lymphomes (lymphome folliculaire, lymphome diffus, Lymphome de la zone marginale, maladie de Hodgkin), l'aplasie et les leucémies et myélodysplasies (leucémie aiguë lymphomblastique, leucémie aiguë myéloïde, myélodysplasie, aplasie médullaire), les syndromes myéloprolifératifs (myélofibrose, polyglobulie primitive, thrombocythémie essentielle, leucémie myéloïde chronique).

Dans un mode de réalisation encore plus avantageux, l'invention concerne un anticorps monoclonal ou polyclonal dirigé contre la protéine BDCA-2 pour son utilisation selon la définition précédente, où ledit anticorps est choisi parmi les anticorps suivants : un anticorps murin, un anticorps chimérique, un anticorps humanisé et un anticorps humain.

Il est bien connu de l'homme de métier que les anticorps murins sont des anticorps produits par des cellules de souris, et les anticorps humains sont produits par des cellules humaines.
Toutefois, on généralisera dans l'invention les définitions précédentes d'anticorps humain et murin à tout anticorps comprenant des séquences d'acides aminés de leurs chaines lourdes et de leurs chaines légères d'homme ou des souris. Aussi, un anticorps murin est un anticorps dont les séquences des chaines lourdes et des chaines légères qui le constituent sont des séquences dont on retrouve la correspondance en acide nucléique dans le génome des cellules B murines. Cet anticorps est donc constitué de séquences d'acides aminés murines, quelle que soit l'origine de la cellule qui permet sa production. Par exemple, les séquences d'anticorps de souris exprimées dans des cellules de macaque donneront des anticorps murins.
La définition ci-dessus s'applique mutatis mutandis aux anticorps humains.

Par anticorps chimérique, on entend dans l'invention un anticorps isolé, dans lequel la séquence de chaque chaîne légère et/ou de chaque chaîne lourde qui le constitue comprend ou consiste en une séquence hybride issue d'au moins deux animaux distincts. Notamment les anticorps chimériques de l'invention sont des hybrides homme/macaque ou homme/souris, ce qui signifie qu'une région de la séquence des chaînes légères et des chaînes lourdes est issue de la séquence d'une immunoglobuline de macaque ou de souris, et que le reste de la séquence desdites chaînes lourdes et des dites chaînes légères est issue de la séquence d'une, ou éventuellement plusieurs, immunoglobuline humaine. Par anticorps humanisé, on entend dans l'invention un anticorps issus d'un animal autre que l'homme dans lequel les séquences des chaines lourdes et des chaines légères autres que les CDR ont été remplacées par des séquences correspondantes d'un ou plusieurs anticorps d'origine humaines. L'anticorps est donc majoritairement constitué de séquences humaines, mais sa spécificité pour l'antigène conférée par les CDR est issue d'une autre espèce.

Dans encore un autre mode de réalisation avantageux, l'invention concerne un anticorps monoclonal ou polyclonal dirigé contre la protéine BDCA-2 pour son utilisation susmentionnée, les tumeurs sont des tumeurs solides choisies parmi le cancer du poumon, rein, foie, pancréas, le mélanome et l'ovaire.

Dans encore un autre mode de réalisation avantageux, l'invention concerne un anticorps monoclonal ou polyclonal dirigé contre la protéine BDCA-2 pour son utilisation susmentionnée, où ledit anticorps est un anticorps chimérique et de préférence un anticorps chimérique choisi parmi un anticorps chimérique murin/humain ou un anticorps chimérique humain/macaque.

Dans encore un autre mode de réalisation avantageux, l'invention concerne un anticorps monoclonal ou polyclonal dirigé contre la protéine BDCA-2 pour son utilisation susmentionnée, où ledit anticorps est un anticorps humanisé.

Dans encore un autre mode de réalisation avantageux, l'invention concerne un anticorps monoclonal ou polyclonal dirigé contre la protéine BDCA-2 pour son utilisation susmentionnée, où ledit anticorps est un anticorps humain.

L'invention concerne un anticorps monoclonal ou polyclonal dirigé contre la protéine BDCA-2 pour son utilisation susmentionnée, où ledit anticorps présente un taux de fucosylation inférieur à 60% des formes glycosylées.

Les anticorps selon l'invention présentent une faible quantité de fucose sur les chaines glycanniques portées par lesdits anticorps. Cette quantité de fucose, ou taux de fucose, est défini comme la proportion moyenne de fucose portée par tous les anticorps, par rapport à la quantité maximale de fucose que peuvent porter les chaines glycanniques.

Un autre objet de l'invention est un anticorps, où ledit anticorps est couplé à une molécule bioactive choisi parmi les radio-isotopes, les métaux non-radioactifs, les toxines, les acides nucléiques, les agents cytotoxiques ou encore les enzymes. L'invention concerne de plus un anticorps monoclonal tels que définis précédemment, où ledit anticorps est couplé à une molécule bioactive choisi parmi les radio-isotopes, les métaux non-radioactifs, les toxines (choisies parmi la ricine, l'abrine, la toxine diphtérique), les acides nucléiques choisis parmi les ARNs anti-sens, les agents cytotoxiques choisis parmi la mitomycine C, le méthotrexate, l'adriamycine, les enzymes telles que les RNases, la biotine, l'avidine ou la streptavidine.
De tels anticorps couplés à une molécule bioactive sont capable d'adresser spécifiquement un radio isotope, une enzyme, un métal lourd, ou encore une toxine, greffé audit anticorps à une cellule cible déterminée, en l'occurrence les cellules DC plasmacytoïdes.
On peut citer comme isotopes avantageux les radio-isotopes suivants At211, I131, I125, Y90, Re186, Re188, Sm153, Bi212, P32, cette liste n'étant pas limitative.
Les toxines que l'on peut greffer aux anticorps selon l'invention sont choisies parmi la ricine, la toxine tétanique, l'abrine, la toxine diphtérique.
Les agents cytotoxiques choisis parmi les antifolates (méthotrexate, pémétrexed, raltitrexed) les anti-purines (cladribine, fludarabine, azathioprine, azathioprine, mercaptopurine, 5-fluorouracile capécitabine, cytarabine, gemcitabine), les inhibiteurs de topoisomérases I et II, les agents alkylants (chlorméthine, cyclophosphamide, ifosfamide, carmustine, fotémustine) et apparentés (mitomycine C, cisplatine, carboplatine, oxaliplatine), les agents intercalants, les anthracyclines (daunorubicine, doxorubicine et chlorydrate, epirubicine, idarubicine, bléomycine), les taxanes, les inhibiteurs spécifiques de tyrosine-kinase (imatinib, Erlotinib), peuvent également être utilisés. L'ensemble de ces molécules ou composés que l'on peut greffer sur les anticorps selon l'invention sont bien connus de l'homme de métier, et il lui est aisé de déterminer la molécule à utiliser.

### LEGENDE DES FIGURES

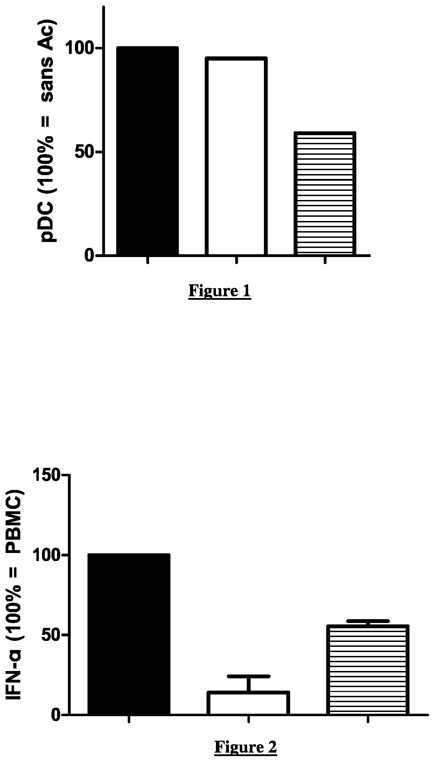

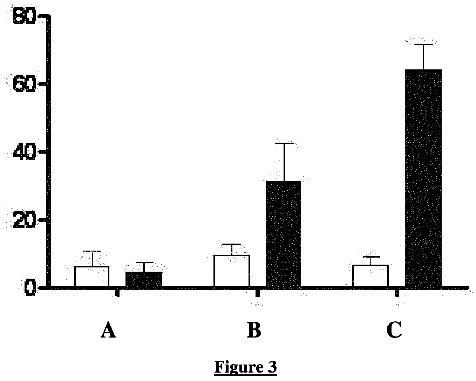

**La** **figure 1** représente un histogramme montrant la déplétion des cellules DC plasmacytoïdes BDCA-2+ dans des PBMC humaines de donneur sain en présence de l'anticorps polyclonal de lapin anti-BDCA-2+. Le % de cellules positives est obtenu par un double marquage ILT7/ CD123.
   La colonne noire représente le pourcentage de cellule cellules DC plasmacytoïdes sans traitement avec l'anticorps (contrôle négatif 1), la colonne blanche représente le pourcentage de cellule cellules DC plasmacytoïdes traitées avec un anticorps contrôle (qui n'est pas dirigé contre BDCA-2 ; contrôle négatif 2) et la colonne hachurée représente le pourcentage de cellule cellules DC plasmacytoïdes traitées l'anticorps anti BDCA-2.
**La** **figure 2** représente un histogramme montrant le dosage de l'IFN-a après déplétion des cellules DC plasmacytoïdes avec un anticorps polyclonal de lapin anti-BDCA-2+ et activation par du CpG.
   La colonne noire représente le pourcentage de sécrétion d'IFN-a par les PBMC totaux, la colonne blanche représente le pourcentage de sécrétion d'IFN-a par les PBMC totaux dépélété en cellules DC plasmacytoïdes et la colonne hachurée représente le pourcentage de sécrétion d'IFN-a par les cellules DC plasmacytoïdes seules.
**La** **figure 3** représente un histogramme montrant l'activité ADCC de l'anticorps polyclonal anti-BDCA-2 (barres noires) sur les cellules Jurkat-BDCA-2. Les résultats sont exprimés en pourcentage de lyse de la cellule Jurkat-BDCA-2 en fonction de la quantité d'anticorps ajoutée, exprimé en facteur de dilution de la solution initiale d'anticorps (A: 0, B: 1/250 et C: 1/25). Moyenne +/- écart type. En contrôle, le pourcentage de lyse est mesuré à l'aide d'un anticorps contrôle (barres blanches).
   L'axe des Y représente le pourcentage de lyse.

### EXEMPLES

### Exemple 1 : préparation de lignées cellulaires exprimant la protéine BDCA-2.

L'ADN complémentaire de BDCA-2 est amplifié par PCR et cloné clonée dans le vecteur d'expression pcDNA3.1(-) aux sites de restriction HindIII/BamHI.
Le vecteur pcDNA contenant BDCA-2 est transfecté par nucléofection ou Fugene HD la lignée cellulaire U937 ou THP-1.

48h heures après transfection, l'expression de BDCA-2 par les cellules transfectées est suivie par cytométrie en flux, en utilisant un anticorps anti-BDCA-2 et un anticorps couplé à un fluorophore.
Les cellules BDCA-2+ seront triées par sélection positive à l'aide de billes magnétiques couplées à l'anticorps anti-BDCA-2 (kit myltenyi).
Le tri est répété jusqu'à obtention de 100% de cellules positives exprimant BDCA-2.

### Exemple 2 : Mesure de l'effet de l'anticorps anti-BDCA-2 selon l'invention.

L'effet de l'anticorps anti-BDCA-2 selon l'invention est mesuré chez la souris ou chez l'homme selon sa nature.
Un moyen de mesurer l'activité de l'anticorps est de mesurer la lyse cellulaire dépendante des anticorps (ADCC) en présence de cellules tueuses (Natural Killer (NK) ou lignées T transfectées avec des récepteurs Fc).

### Mesure de l'ADCC pour un anticorps murin ou présentant un fragment Fc d'origine murine

Les cellules tueuses (cellules effectrices de souris ou des lignées T transfectées avec des FcR murins) sont incubées avec des cellules cibles transfectées BDCA-2 (exemple 1) ou des cellules dendritiques plasmacytoïdes de patients leucémiques (CD4+/CD56+) ou atteints de maladie inflammatoire et/ou auto-immune, dans un ratio E/T de 15/1, en présence de différentes concentrations d'anticorps anti-BDCA-2.

Après 4 ou 16 heures d'incubation, l'activité cytotoxique induite par les anticorps anti-BDCA-2 est mesurée par colorimétrie en dosant dans les surnageants la libération de la lactate déshydrogénase (LDH) (Roche Diagnostics - Cytotoxicity Detection Kit LDH réf 11644793001).

En cas de faible taux de LDH intra-cellulaire, les cellules sont marquées avec un agent fluorescent et la lyse est estimée en mesurant la quantité de fluorescence relarguée dans le surnageant.

Les résultats de lyse spécifique sont exprimés en pourcentage de lyse en fonction de la concentration d'anticorps. Les valeurs d'EC₅₀ (quantité d'anticorps induisant 50% de la lyse maximale) ainsi que les valeurs d'Eₘₐₓ (pourcentage de lyse maximal) sont calculées à l'aide du logiciel PRISM.

### Mesure de l'ADCC pour un anticorps possédant un fragment Fc d'origine humaine

Les cellules tueuses (PBMC, cellules NK, ou cellules T transfectées avec du CD16 humain) sont préalablement purifiées par la technique de déplétion négative développée par la société Miltenyi (Miltenyi Biotec - NK cell isolation kit human réf 130-092-657), à partir de sang périphérique de donneurs sains. La technique ADCC consiste à incuber les cellules NK avec des cellules cibles transfectées BDCA-2 ou des cellules DC plasmacytoïdes de patients leucémiques (CD4+/CD56+) ou atteints de maladie inflammatoire et/ou auto-immune, à un ratio E/T de 15/1, en présence de différentes concentrations d'anticorps anti-BDCA-2.

Après 4 ou 16 heures d'incubation, l'activité cytotoxique induite par les anticorps anti-BDCA-2 est mesurée par colorimétrie en dosant dans les surnageants, une enzyme intracellulaire nommée lactate déshydrogénase (LDH) libérée par les cellules cibles lysées (Roche Diagnostics - Cytotoxicity Detection Kit LDH réf 11644793001).

En cas de faible taux de LDH intra-cellulaire, les cellules sont marquées avec un agent fluorescent et la lyse est estimée en mesurant la quantité de fluorescence relarguée dans le surnageant.

Les résultats de lyse spécifique sont exprimés en pourcentage de lyse en fonction de la concentration d'anticorps. Les valeurs d'EC₅₀ (quantité d'anticorps induisant 50% de la lyse maximale) ainsi que les valeurs d'Eₘₐₓ (pourcentage de lyse maximal) sont calculées à l'aide du logiciel PRISM.

### Exemple 3 : préparation de lignées cellulaires exprimant la protéine BDCA-2.

L'ADN complémentaire de BDCA-2 est amplifié par PCR et cloné clonée dans le vecteur d'expression pcDNA3.1(-) aux sites de restriction HindIII/BamHI. Le vecteur pcDNA contenant BDCA-2 est transfecté par nucléofection ou Fugene HD la lignée cellulaire Jurkat. 48h heures après transfection, l'expression de BDCA-2 par les cellules transfectées est suivie par cytométrie en flux, en utilisant un anticorps anti-BDCA-2 couplé à un fluorophore. Les cellules BDCA-2+ seront isolées à l'aide de tris successifs réalisés par cytométrie en flux avec un trieur (Altra, Becton Dickson) en utilisant un anticorps anti BDCA-2 (Myltenyi). Le tri est répété jusqu'à obtention de 100% de cellules positives exprimant BDCA-2.

### Exemple 4 : Cytotoxicité.

### Déplétion de cellules DC plasmacytoïdes dans des PBMC

Les PBMC sont isolés à partir du sang périphérique sur un gradient de Ficoll. La technique consiste à incuber les PBMC en présence d'un anticorps polyclonal de lapin anti BDCA-2 ou d'anticorps polyclonal de lapin irrelevant. Après 16 heures d'incubation, la dépletion des cellules DC plasmacytoïdes induite par les anticorps anti BDCA-2 est mesurée par cytométrie en flux sur la base du double marquage ILT7/CD123.
Les résultats sont montrés dans la figure 1.
Ces résultats montrent une diminution du nombre de cellules DC plasmacytoïdes d'environ 40% en présence d'anticorps anti-BDCA-2 en comparaison à l'anticorps contrôle. Ce résultat permet de conclure qu'un anticorps anti-BDCA-2, en présence de cellules effectrices, peut induire une déplétion des cellules DC plasmacytoïdes.

### Diminution du taux d'IFNα associé à la déplétion des cellules DC plasmacytoïdes

Les PBMC sont isolés à partir du sang périphérique sur un gradient de Ficoll. La technique consiste à dépléter les cellules DC plasmacytoïdes des PBMC à l'aide du kit Myltenyi anti-cellules DC plasmacytoïdes. Après activation par CpG pendant 16h à 37°C, le taux d'IFN-a est mesuré par cytométrie en flux et cela en comparaison avec les PBMC témoin contenant des cellules DC plasmacytoïdes.
Les résultats sont montrés dans la figure 2.
Les résultats indiquent une diminution de la sécrétion d'IFN-a après déplétion des cellules DC plasmacytoïdes d'environ 85%, ceci permettant de conclure que la déplétion des cellules DC plasmacytoïdes par un anti-BDCA-2 peut conduire à une diminution de la sécrétion d'IFN-a. Dans cette expérience, les cellules DC plasmacytoïdes seules sont utilisées en tant que cellules positives pour la sécrétion d'IFN-a.

### ADCC

Les cellules tueuses (cellules NK) sont préalablement purifiées par la technique de déplétion négative développée par la société Miltenyi (Miltenyi Biotec - NK cell isolation kit human réf 130-092-657), à partir de sang périphérique de donneurs sains. La technique ADCC consiste à incuber les cellules NK avec des cellules cibles de la lignée Jurkat transfectées avec le récepteur BDCA-2, en présence de différentes concentrations d'un anticorps polyclonal de lapin anti-BDCA-2 ou d'anticorps polyclonal de lapin irrelevant. Après 16 heures d'incubation, l'activité cytotoxique induite par les anticorps anti-BDCA-2 est mesurée par colorimétrie en dosant dans les surnageants, une enzyme intracellulaire nommée lactate déshydrogénase (LDH) libérée par les cellules cibles lysées (Roche Diagnostics - Cytotoxicity Detection Kit LDH).

Les résultats sont présentés dans la figure 3 et montre une activité cytotoxique de l'anticorps polyclonal de lapin anti-BDCA-2 sur les cellules Jurkat-BDCA-2, de manière dose dépendante pouvant atteindre plus de 60% de lyse de la cellule cible.

Ces résultats indiquent donc qu'en ciblant l'antigène BDCA-2 exprimé à la surface d'une cellule, il est possible de lyser cette cellule en utilisant des effecteurs tels que les cellules NK. L'anticorps anti-BDCA-2 peut supporter d'autres activités cytotoxiques telles que la phagocytose dépendante d'autres cellules effectrices comme les macrophages ou les neutrophiles.

## Revendications

1. Composition d'anticorps monoclonaux dirigés contre la protéine BDCA-2, lesdits anticorps présentant un taux de fucosylation inférieur à 60% des formes glycosylées, et lesdits anticorps impliquant une destruction des cellules DC plasmacytoïdes par le mécanisme de l'ADCC.

2. Composition d'anticorps monoclonaux dirigés contre la protéine BDCA-2 selon la revendication 1, où lesdits anticorps sont choisis parmi les anticorps suivants : les anticorps murins, les anticorps chimériques, les anticorps humanisés et les anticorps humains.

3. Composition d'anticorps monoclonaux dirigés contre la protéine BDCA-2 selon la revendication 2, où lesdits anticorps sont des anticorps chimériques et de préférence des anticorps chimériques choisis parmi les anticorps chimériques murin/humain ou les anticorps chimériques humain/macaque.

4. Composition d'anticorps monoclonaux dirigés contre la protéine BDCA-2 selon la revendication 2, où lesdits anticorps sont des anticorps humanisés.

5. Composition d'anticorps monoclonaux dirigés contre la protéine BDCA-2 selon la revendication 2, où lesdits anticorps sont des anticorps humains.

6. Composition d'anticorps monoclonaux dirigés contre la protéine BDCA-2 selon l'une quelconque des revendications 1 à 5, où lesdits anticorps sont couplés à une molécule bioactive choisie parmi :
- les radio-isotopes,
- les métaux non-radioactifs,
- les toxines, choisies parmi la ricine, l'abrine, la toxine diphtérique,
- les acides nucléiques choisis parmi les ARNs anti-sens,
- les agents cytotoxiques choisis parmi :
∘ les antifolates, choisis parmi méthotrexate, pémétrexed, raltitrexed,
∘ les anti-purines, choisies parmi cladribine, fludarabine, azathioprine, azathioprine, mercaptopurine, 5-fluorouracile capécitabine, cytarabine, gemcitabine,
∘ les inhibiteurs de topoisomérases I et II,
∘ les agents alkylants choisis parmi chlorméthine, cyclophosphamide, ifosfamide, carmustine, fotémustine, et les agents alkylants apparentés choisis parmi mitomycine C, cisplatine, carboplatine, oxaliplatine,
∘ les agents intercalants,
∘ les anthracyclines choisies parmi daunorubicine, doxorubicine et chlorydrate, epirubicine, idarubicine, bléomycine,
∘ les taxanes,
∘ les inhibiteurs spécifiques de tyrosine-kinase choisis parmi imatinib, Erlotinib,
- les enzymes telles que les RNases,
- la biotine, l'avidine ou la streptavidine.

7. Composition d'anticorps monoclonaux dirigés contre la protéine BDCA-2 selon l'une quelconque des revendications 1 à 6, pour son utilisation comme médicament.

## Patentansprüche

1. Zusammensetzung aus monoklonalen Antikörpern, die gegen das BDCA-2-Protein gerichtet sind, wobei die Antikörper eine Fucosylierungsrate von weniger als 60% der glykosylierten Formen aufweisen und die Antikörper eine Zerstörung von DC-Plasmozytenzellen durch den ADCC-Mechanismus beinhalten.

2. Zusammensetzung aus monoklonalen Antikörpern, die gegen das BDCA-2-Protein gerichtet sind nach Anspruch 1, wobei die Antikörper aus den folgenden Antikörpern gewählt sind: murine Antikörper, chimäre Antikörper, humanisierte Antikörper und humane Antikörper.

3. Zusammensetzung aus monoklonalen Antikörpern, die gegen das BDCA-2-Protein gerichtet sind nach Anspruch 2, wobei die Antikörper chimäre Antikörper und vorzugsweise chimäre Antikörper sind, die aus chimären murinen/humanen Antikörpern oder chimären humanen/Makaken Antikörpern gewählt sind.

4. Zusammensetzung aus monoklonalen Antikörpern, die gegen das BDCA-2-Protein gerichtet sind nach Anspruch 2, wobei die Antikörper humanisierte Antikörper sind.

5. Zusammensetzung aus monoklonalen Antikörpern, die gegen das BDCA-2-Protein gerichtet sind nach Anspruch 2, wobei die Antikörper menschliche Antikörper sind.

6. Zusammensetzung aus monoklonalen Antikörpern, die gegen das BDCA-2-Protein gerichtet sind nach einem der Ansprüche 1 bis 5, wobei die Antikörper an ein bioaktives Molekül gekoppelt sind, gewählt aus:
- Radioisotopen,
- nicht-radioaktiven Metallen,
- Toxinen, gewählt aus Ricin, Abrin, Diphtherietoxin,
- Nukleinsäuren, gewählt aus Antisense-RNAs,
- zytotoxische Mittel, gewählt aus:
∘ Antifolaten, gewählt aus Methotrexat, Pemetrexed, Raltitrexed,
∘ Anti-Purinen, gewählt aus Cladribin, Fludarabin, Azathioprin, Azathioprin, Mercaptopurin, 5-Fluorouracil, Capecitabin, Cytarabin, Gemcitabin,
∘ Topoisomerase I und II Inhibitoren,
∘ Alkylierungsmitteln, gewählt aus Chlormethin, Cyclophosphamid, Ifosfamid, Carmustin, Fotemustin und verwandten Alkylierungsmitteln, gewählt aus Mitomycin C, Cisplatin, Carboplatin, Oxaliplatin,
∘ Interkalationsmitteln,
∘ Anthracyclinen, gewählt aus Daunorubicin, Doxorubicin und Chlorhydrat, Epirubicin, Idarubicin, Bleomycin,
∘ Taxanen,
∘ spezifischen Tyrosinkinase-Inhibitoren gewählt aus Imatinib, Erlotinib,
- Enzyme wie RNasen,
- Biotin, Avidin oder Streptavidin.

7. Zusammensetzung aus monoklonalen Antikörpern, die gegen das BDCA-2-Protein gerichtet sind nach einem der Ansprüche 1 bis 6, zur Verwendung als Medikament.

## Claims

1. Composition of monoclonal antibodies directed against the BDCA-2 protein, said antibodies having a fucosylation level of less than 60% of the glycosylated forms, and said antibodies involving destruction of plasmacytoid DC cells by the ADCC mechanism.

2. Composition of monoclonal antibodies directed against the BDCA-2 protein according to claim 1, where said antibodies are chosen from the following antibodies: murine antibodies, chimeric antibodies, humanised antibodies, and human antibodies.

3. Composition of monoclonal antibodies directed against the BDCA-2 protein according to claim 2, where said antibodies are chimeric antibodies and preferably chimeric antibodies chosen from murine/human chimeric antibodies or human/macaque chimeric antibodies.

4. Composition of monoclonal antibodies directed against the BDCA-2 protein according to claim 2, where said antibodies are humanised antibodies.

5. Composition of monoclonal antibodies directed against the BDCA-2 protein according to claim 2, where said antibodies are human antibodies.

6. Composition of monoclonal antibodies directed against the BDCA-2 protein according to any one of claims 1 to 5, where said antibodies are coupled to a bioactive molecule chosen from:
- radio-isotopes,
- non-radioactive metals,
- toxins, chosen from ricin, abrin, diphtheria toxin,
- nucleic acids chosen from anti-sense RNAs,
- cytotoxic agents chosen from:
∘ antifolates, chosen from methotrexate, pemetrexed, raltitrexed,
∘ anti-purines, chosen from cladribine, fludarabine, azathioprine, azathioprine, mercaptopurine, 5-fluorouracil capecitabine, cytarabine, gemcitabine,
∘ inhibitors of topoisomerases I and II,
∘ alkylating agents chosen from chlormethine, cyclophosphamide, ifosfamide, carmustine, fotemustine, and related agents chosen from mitomycin C, cisplatin, carboplatin, oxaliplatin,
∘ intercalating agents,
∘ anthracyclines chosen from daunorubicin, doxorubicin and chlorydrate, epirubicin, idarubicin, bleomycin,
∘ taxanes,
∘ tyrosine-kinase specific inhibitors chosen from imatinib, Erlotinib,
- enzymes such as RNases,
- biotin, avidin or streptavidin.

7. Composition of monoclonal antibodies directed against the protein BDCA-2 according to any one of claims 1 to 6, for use as a drug.
